# EUROPEAN PATENT SPECIFICATION

(11) **EP 0 365 676 B1**
(45) Date of publication and mention of the grant of the patent: **14.04.1993**
(21) Application number: 87906471.5
(22) Date of filing: 06.10.1987
(51) Int. Cl.: A61M 1/22, A61M 1/34

(54) **BLOOD COMPONENT SEPARATOR**
ABTRENNVORRICHTUNG EINES BLUTBESTANDTEILES
SEPARATEUR DES COMPOSANTS SANGUINS

(30) Priority: 06.10.1986 JP 237606/86
(43) Date of publication of application: 02.05.1990
(73) Proprietor: TERUMO KABUSHIKI KAISHA, Tokyo 151 (JP)
(72) Inventor: NAOI, Keiji, 2656-1, Ohbuchi Fuji-shi Shizuoka 417 (JP); IWATA, Katsuhiko, 2656-1, Ohbuchi Fuji-shi Shizuoka 417 (JP)
(74) Representative: Henkel, Feiler, Hänzel & Partner
(86) International application number: JP8700749
(87) International publication number: WO8802264

(56) References cited:
- DE-A- 2 148 670
- GB-A- 1 520 992
- JP-A- 5 940 044
- JP-A-51 111 960
- JP-B- 605 309
- US-A- 3 765 536

## Description

### Technical Field:

This invention relates to an apparatus for the separation of blood components. More particularly, it relates to an apparatus for the separation of blood components, which enables the removal of leukocyte from a hemocyte suspension such as the concentrated red cell to be effected by a simple procedure efficiently in terms of both quantity and time, and that with a remote possibility of entailing leakage of filter fiber fragments in the blood component selected for transfusion.

### Background Art:

In recent years, the progress and dissemination of flexible blood bags has greatly encouraged popular acceptance of the method of therapy which resorts to exclusive transfusion of a particular blood component required by a patient under care. For the purpose of this component transfusion, the blood obtained from a donor is separated by a centrifugal treatment into concentrated red cells(CRC), platelet concentrate (PC), platelet poor plasma (PPP), etc.

The concentrated red cells thus separated is extensively used as a componental preparation of erythrocytes for exclusive transfusion to patients requiring supply of erythrocytes. Actually, however, the concentrated red cells richly contains leukocytes and platelets. In the circumstance, the concept of this liquid as the so-called total component blood has been gaining general approval. Thus, great anxiety is expressed about the fact that the transfusion of the concentrated red cell to a patient requiring supply of only hemocyte inevitably results in the unnecessary transfusion of large amounts of leukocyte and platelets.

Among means for removing leukocytes and platelets from hemocyte suspensions such as the concentrated red cell, there is counted a method which effects the removal by adding an erythrocyte agglutinant such as dextran or hydroxyethylstarch into a leukocyte containing suspension as CRC and then subjecting the agglutinant incorporated liquid to sedimentation to separate the liquid into an erythrocyte sediment and a leukocyte-containing liquid layer. This method, however, has a disadvantage that it takes up much time and labor and the removal of leukocytes is obtained with poor efficiency. At present a method which comprises passing the hemocyte suspension through a column packed with fibers thereby causing the leukocytes in the suspension to be adsorbed on the fibers and thus removed from the suspension. In this method, though the ratio of removal of leukocytes and platelets can be heightened by increasing the packing density of fibers, the increased packing density results in an addition to the time of filtration of the liquid. Thus, it has been difficult for this method to satisfy the ratio of removal of erythrocytes and the time required for the treatment of the hemocyte suspension at the same time. For the solution of this difficulty, there have been proposed a method which uses extremely thin fibers by themselves as a filter material and a method which uses a non-woven fabric made of such extremely thin fibers as a filter material (Japanese Patent Publication SHO 58(1983)-54,126 and Japanese Patent Laid-Open SHO 60(1985)-193,468). Where the extremely thin fibers themselves are packed, the aforementioned problem on the balance between the ratio of removal of hemocytes and the time for the treatment cannot be completely solved and the packing of fibers possibly entails the phenomenon of channeling, depending on the manner of packing, and the fibers have the possibility of undergoing fragmentization and consequently leaking in the filtrate. Where these extremely thin fibers are used in the form of a non-woven fabric, unless the component fibers are mutually fastened by heating or with an adhesive agent, the fibers in the insufficiently intertwined state have the possibility of undergoing fragmentization and leaking in the filtrate.

GB-A-1 520 992 discloses an apparatus for the separation of blood components comprising a housing provided with an inlet for a hemocyte suspension and an outlet for said hemocyte suspension as well as at least one filter medium acting as a sieve within said housing. By the said filter medium there are to be removed undesired debris like blood clots and various aggregates of leukocyte or platelet type from blood. Said filter medium should not be made from multifilament or spun yarns due to their deleterious effects on blood.

An object of this invention, therefore, is to provide an improved apparatus for the separation of blood components.

Another object of this invention is to provide an apparatus for the separation of blood components. More particularly, this invention relates to an apparatus for the separation of blood components, which enables the removal of leukocytes and platelets from a hemocyte suspension such as the concentrated red cells to be effected by a simple procedure efficiently in terms of both quantity and time, and that with a remote possibility of entailing leakage of filter fiber fragments in the blood component selected for transfusion.

### Disclosure of the Invention:

The objects described above are accomplished by an apparatus for the separation of blood components, comprising a housing provided with an inlet for a hemocyte suspension and an outlet for the hemocyte suspension and at least one filter material packed in the housing characterised in that said filter material is selected from the group consisting of woven fabrics and knitted fabrics both made of yarns obtained by twisting a plurality of fibers substantially incapable of degenerating said hemocyte suspension packed in the housing wherein the filter material is packed in a bulk density in the range of 0.2 to 1.0 g/cm³ and wherein the openings in the woven fabrics or knitted fabrics are large enough to permit passage of erythrocytes. Advantageously the openings in the woven fabrics or knitted fabrics have diameters approximately in the range of 10 to 200 µm. This invention also discloses an apparatus for the separation of blood components, wherein the fibers are synthetic fibers, semi-synthetic fibers, regenerated fibers, inorganic fibers, or natural fibers. This invention further discloses an apparatus for the separation of blood components, wherein the fibers are selected from the group consisting of polyamide type, polyester type, polyacrylonitrile type, polystyrene type, polyolefin type, polyurethane type, and acetate type fibers. This invention discloses an apparatus for the separation of blood components, wherein the woven fabrics or knitted fabrics are formed of yarns of fibers having diameters approximately in the range of 10 to 50 µm. This invention also discloses an apparatus for the separation of blood components, wherein the woven fabrics or knitted fabrics are formed of yarns of fibers having diameters approximately in the range of 10 to 30 µm. This invention further discloses an apparatus for the separation of blood components, wherein the openings in the woven fabrics or knitted fabrics have diameters approximately in the range of 10 to 100 µm.

### Brief Description of the Drawings:

Fig. 1 is a cross section of a typical apparatus for the separation of blood components as one embodiment of the present invention, and
Fig. 2 is diagram illustrating a path of treatment incorporating therein the apparatus for the separation of blood components according to the present invention.

### Best Mode for Carrying Out the Invention:

The apparatus for the separation of blood components according to the present invention is characterized by using, as a filter material, either a woven fabric or a knitted fabric made of yarns obtained by twisting fibers which are substantially incapable of degenerating the hemocyte suspension. The woven fabric or knitted fabric possesses "openings" where their component fibers intersect each other and the "openings" serve as courses for the flow of the suspension when the fabric is used as a filter material. In the case of the woven fabric or knitted fabric, these "openings" are arrayed orderly. Even when a multiplicity of such woven fabrics or knitted fabrics are superposed and compressed (for addition of packing density), therefore, these openings are retained intact. Thus, the apparatus for the separation of blood components functions advantageously as means for rapidly passing a hemocyte suspension such as the concentrated red cell.

When fibers are used in the form of woven fabrics or knitted fabrics, the packing can be effected more efficiently to a higher density and the efficiency of removal of unnecessary hemocyte in a fixed volume is also higher than when the fibers are used as randomly packed.

The mechanism responsible for the seizure of leukocytes and platelets by the fibers may be logically explained by a supposition that this seizure is caused by the adhesion of leukocytes and platelets to the fibers and the fast deposition of leukocytes and platelets in the gaps between the adjacent fibers. Japanese Patent Publication SHO 58(1983)-54,126 teaches use of extremely thin fibers for the purpose of decreasing the distance of gaps between the adjacent fibers and increasing the surface area of fibers in a fixed volume and consequently heightening the efficiency of seizure. As means of shortening the distance of gaps between adjacent fibers, enlarging the surface area of fibers, and improving the efficiency of seizure, there may be conceived, besides the aforementioned method which resorts to use of such extremely thin fibers, a method for compressing the filter material thereby heightening the packing density. When the fibers themselves are packed and then compressed to increase the packing density, however, the speed of filtration is lowered so much that the apparatus for the separation of blood components will no longer function satisfactorily. It has been held that this phenomenon occurs because the fibers themselves are packed at random and, therefore, allowed to retain courses intact for the flow of the hemocyte suspension only with great difficulty. The inventors took notice of woven fabrics or knitted fabrics of fibers considered to retain the courses for the flow of the hemocyte suspension more easily. As the result, we have perfected the present invention as described above.

Now, the present invention will be described in detail below with reference to a preferred embodiment thereof.

The apparatus for the separation of blood components according to the present invention is characterized by using, as packed in a housing, at least one filter material selected from the group consisting of woven fabrics and knitted fabrics both made of yarns obtained by twisting fibers substantially incapable of degenerating the hemocyte suspension.

The material of the fibers of which the woven fabrics or the knitted fabrics are formed is not specifically restricted except for the sole requirement that it should be substantially incapable of degenerating the hemocyte suspension. Examples of the fibers usable in this invention include synthetic fibers such as the polyamide type, polyester type, polyacrylonitrile type, polystyrene type, polyolefin type, and polyurethane type fibers, semi-synthetic fibers such as the acetate type fibers, regenerated fibers such as the cuprammonium cellulose, inorganic fibers such as the glass fibers, and natural fibers such as the cotton, silk, and wool. Among other fibers enumerated above, the polyamide type, polyester type, polyacrylonitrile type, polystyrene type, polyolefin type, polyurethane type, and acetate type fibers prove to be particularly desirable.

The openings in the woven fabric or knitted fabric to be formed of fibers of the material described above are required to have sufficient flow velocity and chance to contact with the hemocyte suspension during the course of passage of the hemocyte suspension through the filter. Where the hemocyte suspension to be passed through the filter material contains erythrocytes richly, the openings are desired to be capable of sufficiently passing erythrocyte. To be specific, the openings are desired to have diameters approximately in the range of 10 to 200 µm, preferably 10 to 100 µm. The expression "capable of passing erythrocyte" as used herein embraces the case in which erythrocytes can be passed with deformation.

The thickness of the yarns of fibers of which the woven fabric or knitted fabric is not specifically restricted except for the requirement that it should be capable of imparting the aforementioned size to the openings. With a view to ensuring the ease of production of the woven fabric or knitted fabric and to heightening the packing density, the thickness of the yarns generally falls in the range of 200 to 1,500 µm, preferably 200 to 1,000 µm. The yarns of fibers are those formed of a plurality of filaments. The component monofilaments have a thickness in the range of 10 to 50 µm, preferably 10 to 30 µm.

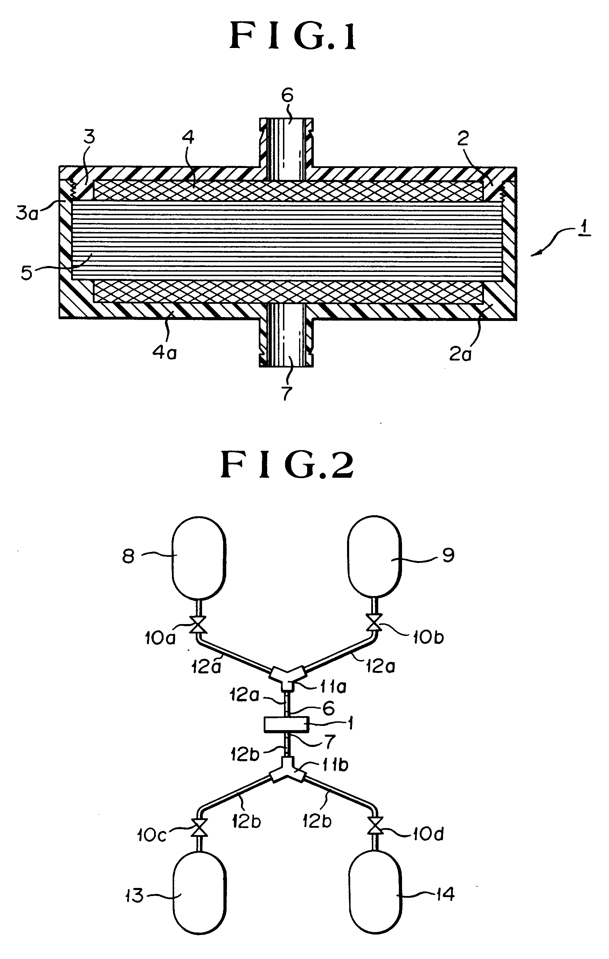

Fig. 1 depicts a typical apparatus for the separation of blood components as one preferred embodiment of this invention. In this preferred embodiment, an apparatus 1 for the separation of blood components comprises disclike housing members 2, 2a watertightly fitted to each other along peripheral fitting parts 3,3a so as to form an empty part therein and a filter material formed by the superposition of a plurality of woven fabrics and/or knitted fabrics as nipped between meshed supporting members 4,4a within the empty part. The one housing member 2 is provided at the central part thereof with an inlet 6 for the hemocyte suspension and the other housing member 2a is provided similarly at the central part thereof with an outlet 7 for the hemocyte suspension in such a manner that the inlet 6 and the outlet 7 will communicate with the empty part within the housing. The supporting members 4, 4a are made of a material such as, for example, the polyester and adapted to support the filter material 5 formed of a plurality of woven fabrics and/or knitted fabrics. They are formed in a meshed patten so as to fulfil concurrently the role of a flow course regulator for uniformizing the incoming and outgoing suspension under treatment. In the apparatus for the separation of blood components constructed as described above, the component woven fabrics and/or knitted fabrics of the filter material 5 are superposed and packed in a bulk density approximately in the range of 0.2 to 1.0 g/cm³ , though variable with the kind and thickness of the component fibers. The bulk density of the filter material 5 affects the speed of filteration of the hemocyte suspension under treatment and the efficiency of seizure of leukocytes to a great extent. Suitably, the amount of the filter material 5 to be packed in the housing falls in the range of 3 to 15 g per 100 ml of the hemocyte suspension to be treated.

The apparatus 1 for the separation of blood components constructed as described above is put to use as illustrated in Fig. 2, for example.

A hemocyte suspension bag 8 containing a hemocyte suspension subjected to filtration and a physiological saline solution bag 9 containing physiological saline solution are disposed in an upper position and are connected via clamp 10a, 10b and a three-way connector 11a to the hemocyte suspension inlet 6 of the apparatus 1 for the separation of blood components by means of connection tube 12a. In the meantime, recovery bags 13, 14 which are disposed at a position lower than the apparatus 1 for the separation of blood components are similarly connected via clamp 10c, 10d and a three-way connector 11b to the hemocyte suspension outlet 7 of the apparatus 1 for the separation of blood components by means of a connection tube 12b. Thus, a path of treatment is complete. The treatment of the hemocyte suspension is started by feeding the physiological saline solution from the physiological saline solution bag 9 thereby priming the interior of the path. Then, from the hemocyte suspension bag 8, the hemocyte suspension is passed through the connection tube 12a and introduced into the apparatus 1 for the separation of blood components spontaneously by virtue of gravitational attraction. The hemocyte suspension thus introduced into the apparatus 1 for the separation of blood components is deprived of leukocyte and platelets by the filter material made of woven fabric and/or knitted and consequently is composed mainly of erythrocyte and plasma. The hemocyte suspension of this new composition is passed through the connection tube 12b and recovered in the recovery bag 13. For the purpose of enhancing the recovery ratio of erythrocytes, the physiological saline solution from the physiological saline solution bag 9 is passed through connection tube 12a and introduced into the apparatus 1 for the separation of blood components, so that part of erythrocytes remaining within the apparatus 1 for the separation of blood components will be entrained by the incoming physiological saline solution and recovered via the connection tube 12b into the other recovery bag 14. By this simple procedure, separation and removal of leukocytes and paltelets can be attained efficiently and rapidly. Examples of the hemocyte suspension to which the operation for the removal of leukocytes and platelets by the use of the apparatus of the present invention include blood, body fluids such a the abdominal dropsy and the medullary fluid, hemocytes suspensions such as the concentrated red cell obtainable by centrifugal separation, hemocyte suspensions obtainable by an agglutiant incorporation-sedimentation process, and hemocytes suspension obtainable by the electrophoresis of cells.

Now, the present invention will be described more specifically below with reference to working examples.

### Example 1

In the empty parts of a housing (56 mm in inside diameter and 21 mm in filter layer length), 70 knitted fabrics formed of yarns of 20 tex produced from polyester filaments of 12 µm in diameter were superposed and packed in a bulk density of 0.5 g/cm³, to complete an apparatus for the separation of blood components as illustrated in Fig. 1. The apparatus for the separation of blood components constructed as described above was set in place 750 mm below a hemocyte suspension bag 8 containing 200 ml of CPD-added blood. A recovery bag 13 (produced by Terumo Kabushiki kaisha and marketed under trademark designation of "Transfer Bag T-200") was set in place 750 mm below the apparatus. They were interconnected with tubes of vinyl chloride resin 3 mm in inside diameter band 6 mm in outside diameter to complete a path of treatment as illustrated in Fig. 2. From a physiological saline solution bag 9, about 100 ml of physiological saline solution was fed to prime the interior of the path and then the blood was allowed to flow down into the apparatus for the separation of blood components by virtue of gravitational attraction. Then, recovery of the erythrocytes was carried out with about 100 ml of physiological saline solution In this operation, the time required for the treatment of blood was 2 minutes 45 seconds, the speed of treatment per unit area was 3.0 ml/cm²/minute, the ratio of removal of leukocytes was 98.0%, and the recovery ratio of erythrocytes was 95.0%.

The ratio of removal of leukocytes and the recovery ratio of red erythrocytes were determined by taking counts of erythrocytes and leukocytes in the blood before and after passage thereof through the filter with the aid of an automatic blood counter (produced by Ortho Diagnostic Cop. and marketed under product code of "ELT-8" and calculating the absolute amounts of the respective blood components based on the total amount of the blood.

### Example 2

The procedure of Example 1 was repeated to form a path of treatment and treat a blood, except that 90 woven fabrics using yarns of 20 tex produced from polyester filaments of 12 µm in diameter were superposed in a bulk density of 0.69 g/cm³ in a housing. In this operation, the time required for the treatment of blood was 3 minutes, the speed of treatment per unit area was 2.7 ml/cm²/minute, the ratio of removal of leukocytes was 99.0%, and the recovery ratio of erythrocytes was 96.0%.

### Control 1

An apparatus for the separation of blood components similar in shape to the apparatuses of Examples 1 and 2 was obtained by uniformly packing, within the empty part of a housing, polyester filaments of 12 µm in diameter directly to a bulk density of 0.35 g/cm³ (which was the upper limit attainable by human power). Then, the treatment of blood was carried out by repeating the procedure of Example 1 in terms of path and method of treatment. In this operation, the required for the treatment of 4 minutes 30 seconds, the speed of treatment per unit area was 2.0 ml/cm²/minute, the ratio of removal of leukocytes was 62%, and the recovery ratio of erythrocytes was 93%.

### Example 3

The procedure of Example 1 was repeated to form a path of treatment and treat a blood, except that 66 knitted fabrics using yarns of 20 tex produced from natural cotton filaments of an average thickness of 16µm were superposed in a bulk density of 0.5 g/cm³ within a housing. In the operation, the time required for the treatment was 3 minutes, the speed of treatment per unit area was 2.7 ml/cm²/minute, the ratio of removal of leukocytes was 98.3%, and the recovery ratio of erythrocytes was 96.0%.

### Control 2

The procedure of Example 1 was repeated to form a path of treatment and treat a blood, except that natural cotton filaments having an average thickness of 16µm similarly to those of Example 3 were uniformly packed in a bulk density of 0.35 g/cm² (which was the upper limit attainable by human power). In this operation, the time required for the treatment was 7 minutes 30 seconds, the speed of treatment per unit area was 1.1 ml/cm²/minute, the ratio of removal of leukocytes was 94.8%, and the recovery ratio of erythrocytes was 93.2%.

### Industrial Applicability:

As described above, the present invention is directed to an apparatus for the separation of blood components is disclosed which comprises a housing provided with an inlet for a hemocyte suspension and an outlet for the hemocyte suspension and at least one filter material selected from the group consisting of woven fabrics and knitted fabrics both made of yarns obtained by twisting a plurality of fibers substantially incapable of degenerating the hemocyte suspension and packed in the housing. Owing to the construction, the filter material is enabled to retain intact courses for the flow of the hemocyte suspension even when the filter material is packed in a high packing density and the apparatus is enabled to effect the removal of unwanted blood components by a simple procedure with high efficiency in terms of both quality of time. Further, since the yarns of fibers are used in the form of woven fabrics and/or knitted fabrics, the possibility of the fibers undergoing fragmentization and consequently leaking in the filtrate and the possibility of the blood under treatment giving rise to the phenomenon of channeling are very remote.

The apparatus of the present invention for the separation of blood components is such that flow velocity of the blood component becomes sufficient and chance for contacting blood component with fibers becomes sufficient, and as the result the seizure of leukocytes and platelets by the openings in the woven fabrics or knitted fabrics is attained advantageously when the range of 0.2 to 1.0 g/cm³ and the opening in the woven fabrics and/or knitted fabrics are large enough for passage of erythrocytes, specifically having diameters in the range of 10 to 100 µm. Further, when the fibers of which the woven fabrics and/or knitted fabrics are formed are selected from the group consisting polyamido type, polyester type, polyacrylonitrile type, polystyrene type, polyolefin type, polyurethane type, and acetate type fibers, the seizure of leukocytes and platelets by adhesion to the fibers is effected more efficiently and the efficiency of the seizure is proportionately enhanced. When the woven fabrics or knitted fabrics are formed of yarns of fibers having diameters in the range of 10 to 30 µm, the apparatus for the separation of blood components proves to be all the more desirable in terms of ease of production and improvement of packing density.

## Claims

1. An apparatus for the separation of blood components comprising a housing (2,2a) provided with an inlet (6) for a hemocyte suspension and an outlet (7) for said hemocyte suspension and at least one filter material (5) packed in said housing (2,2a), characterized in that said filter material (5) is selected from the group consisting of woven fabrics and knitted fabrics both made of yarns obtained by twisting a plurality of fibers substantially incapable of degenerating said hemocyte suspension, wherein said filter material is packed in a bulk density in the range of 0.2 to 1.0 g/cm³ and wherein the openings in said woven fabrics or knitted fabrics are large enough to permit passage of erythrocytes.

2. An apparatus according to Claim 1, wherein the openings in said woven fabrics or knitted fabrics have diameters approximately in the range of 10 to 200 µm.

3. An apparatus according to Claim 1, wherein said fibers are synthetic fibers, semi-synthetic fibers, regenerated fibers, inorganic fibers, or natural fibers.

4. An apparatus according to Claim 3, wherein said fibers are selected from the group consisting of polyamide type, polyester type, polyacrylonitrile type, polystyrene type, polyolefin type, polyurethane type, and acetate type fibers.

5. An apparatus according to Claim 1, wherein said woven fabrics or knitted fabrics are formed of yarns of fibers having diameters approximately in the range of 10 to 50 µm.

6. An apparatus according to Claim 1, wherein said woven fabrics or knitted fabrics are formed of yarns of fibers having diameters approximately in the range of 10 to 30 µm.

7. An apparatus according to Claim 1, wherein said openings in said woven fabrics or knitted fabrics have diameters approximately in the range of 10 to 100 µm.

## Patentansprüche

1. Vorrichtung zur Auftrennung von Blutkomponenten, umfassend ein Gehäuse (2, 2a) mit einem Einlaß (6) für eine Hämozyten-Suspension und einem Auslaß (7) für die Hämozyten-Suspension sowie mindestens einem in dem Gehäuse (2, 2a) untergebrachten Filtermaterial (5), dadurch gekennzeichnet, daß das Filtermaterial (5) aus der Gruppe Gewebe und Gewirke, beide hergestellt aus durch Verzwirnen mehrerer, zum Degenerieren der Hämozyten-Suspension praktisch unfähiger Fasern erhaltenen Garnen, wobei das Filtermaterial in einer Schüttdichte im Bereich von 0,2 bis 1,0 g/cm³ gepackt ist und die Öffnungen in den Geweben oder Gewirken groß genug sind, um eine Passage von Erythrozyten zu gestatten.

2. Vorrichtung nach Anspruch 1, worin die Öffnungen in den Geweben oder Gewirken Durchmesser etwa im Bereich von 10 bis 200 µm aufweisen.

3. Vorrichtung nach Anspruch 1, worin es sich bei den Fasern um synthetische Fasern, halbsynthetische Fasern, regenerierte Fasern, anorganische Fasern oder natürliche Fasern handelt.

4. Vorrichtung nach Anspruch 3, worin die Fasern aus der Gruppe Fasern vom Polyamid-, Polyester-, Polyacrylnitril-, Polystyrol-, Polyolefin-, Polyurethan- und Acetattyp ausgewählt sind.

5. Vorrichtung nach Anspruch 1, worin die Gewebe oder Gewirke aus Garnen von Fasern mit Durchmessern etwa im Bereich von 10 bis 50 µm hergestellt sind.

6. Vorrichtung nach Anspruch 1, worin die Gewebe oder Gewirke aus Garnen von Fasern mit Durchmessern etwa im Bereich von 10 bis 30 µm hergestellt sind.

7. Vorrichtung nach Anspruch 1, worin die Öffnungen in den Geweben oder Gewirken Durchmesser etwa im Bereich von 10 bis 100 µm aufweisen.

## Revendications

1. Appareil de séparation des composants sanguins, comprenant un boîtier (2,2a) muni d'une entrée (6) pour une suspension d'hémocytes et d'une sortie (7) pour ladite suspension d'hémocytes, et au moins une matière filtrante (5), enfermée dans ledit boîtier (2,2a), choisie parmi des tissus tissés et des tissus tricotés, tous faits de fils obtenus par torsion d'une pluralité de fibres ne risquant pas de provoquer la dégénérescence de ladite suspension d'hémocytes, appareil dans lequel ladite matière filtrante est enfermée avec une masse volumique en vrac comprise entre 0,2 et 1,0 g/cm³ et les jours dans lesdits tissus tissés ou tricotés sont suffisamment grands pour laisser passer les érythrocytes.

2. Appareil selon la revendication 1, dans lequel les jours dans lesdits tissus tissés ou tricotés ont des diamètres approximativement compris entre 10 et 200 µm.

3. Appareil selon la revendication 1, dans lequel lesdites fibres sont des fibres synthétiques, des fibres semi-synthétiques, des fibres régénérées, des fibres minérales ou des fibres naturelles.

4. Appareil selon la revendication 3, dans lequel lesdites fibres sont choisies dans le groupe formé par les fibres du type polyamide, du type polyester, du type polyacrylonitrile, du type polystyrène, du type polyoléfine, du type polyuréthane et du type acétate.

5. Appareil selon la revendication 1, dans lequel lesdits tissus tissés ou tricotés sont faits de fils de fibres ayant des diamètres approximativement compris entre 10 et 50 µm.

6. Appareil selon la revendication 1, dans lequel lesdits tissus tissés ou tricotés sont faits de fils de fibres ayant des diamètres approximativement compris entre 10 et 30 µm.

7. Appareil selon la revendication 1, dans lequel lesdits tissus tissés ou tricotés sont faits de fils de fibres ayant des diamètres approximativement compris entre 10 et 100 µm.
